# EUROPEAN PATENT APPLICATION

(11) **EP 4 768 050 A1**
(43) Date of publication of application: **01.07.2026**
(21) Application number: 24383475.1
(22) Date of filing: 27.12.2024
(51) Int. Cl.: A61L 27/20, A61L 27/36, A61L 27/38

(54) **MICROSPHERES COMPRISING DECELLULARIZED EXTRACELLULAR MATRIX, NATURAL BIOPOLYMER AND CROSSLINKING AGENT AND METHODS FOR MAKING AND USING THE SAME**

(71) Applicant: Fundación Tecnalia Research & Innovation, 20009 Donastia-San Sebastían Gipuzkoa (ES)
(72) Inventor: GARCIA LIZARRIBAR, Andrea Alicia, DONOSTIA - SAN SEBASTIAN - GIPUZKOA (ES); OLALDE, Beatriz, DONOSTIA - SAN SEBASTIAN - GIPUZKOA (ES)
(74) Representative: Balder IP Law, S.L.

(57) **Abstract**

The present disclosure relates generally to microspheres comprising decellularized tissue extracellular matrix, at least a natural biopolymer, and at least a crosslinking agent. In some embodiments, the microsphere comprises biological cells and/or at least a biomolecule. Moreover, the present disclosure also relates to the method of generating said microspheres and the uses thereof.

## Description

### TECHNICAL FIELD

The invention disclosed herein relates generally to microspheres comprising decellularized adipose tissue extracellular matrix, at least a natural biopolymer, and at least a crosslinking agent, methods of making them, and uses thereof for cell and/or biomolecule delivery to repair or restore tissues in a subject or as a disease treatment material.

### STATE OF THE ART

Tissue engineering is a popular and promising method to repair tissue defects caused by trauma, infection, and tumor. The specific approach is to develop artificial tissue by culturing cells on scaffolds. Therefore, ideal scaffolds for tissue engineering should mimic the architecture of natural extracellular matrix (ECM) to provide a 3D microenvironment for cell growth and signal communication, so the cells can maintain their specialized morphologies and functions. Numerous biomaterials for tissue engineering and regenerative medicine are based on the decellularized extracellular matrix (dECM) to promote rapid healing at the implant site, reduce adverse inflammatory responses, and prevent implant rejection From the perspective of cell carriers, various forms of biomaterials have been developed, such as film, hydrogel, and particle. Among them, particles such as microspheres have become an efficient platform due to their advantages, including (1) the simplicity of large-scale fabrication; (2) homeostatic and regular porous structure; (3) sphere size diversity coupled with high specific surface area; (4) controllable release of therapeutic molecule.

Moreover, biodegradable polymers are the most widely used building materials for microspheres. Biodegradable polymers can be classified into natural and synthetic polymers. Collagen, gelatin, chitosan, and alginate are the main natural biodegradable polymers, and polycaprolactone (PCL), poly(lactic acid) (PLA), poly(glycolic acid) (PGA), and poly(lactic-co-glycolic acid) (PLGA) are the main synthetic polymers used in tissue engineering. The problem of microspheres comprising synthetic polymers is that once these microspheres are obtained and cultured in bioreactors in the presence of cells, in order to be used for tissue regeneration, an additional step of separation of the synthetic microspheres from the cells is necessary. In this sense, the development of microspheres comprising natural polymers is therefore of great interest.

The origin of natural polymers can be from animal or plant sources. Those from animals can contain functional groups specific for cell-polymer interactions, while those from plants lack cell-specific patterns. In this sense, polymers from animals can be isolated such as collagen and fibrinogen. To combine the fast-crosslinking properties of alginate and cell-interaction properties of collagen, natural polymer combinations have been used to produce microspheres with electrospray.

Native tissues are composed of a complex mixture of biomolecules that are not present in isolated natural polymers. Decellularized extracellular matrices preserve many of the biomolecules and associated factors present in live tissues providing them with multiple types of cell-specific interaction groups that play a role in adhesion, homeostasis, metabolism and differentiation processes. Therefore, dECM are one of the most complete natural polymers for cell culture purposes.

Solutions made of digested decellularized matrices are usually non-newtonian viscous fluids that can be physically crosslinked with temperature. Low-concentrated solutions are less viscous, but can loss the crosslinking properties, while high concentrated solutions are cross-linkable, but can be too viscous for some manufacturing methods such as microfluidics and electrospray. Nanometric particles of dECM have been produced using electrospray for heart injury regeneration. However, particles are not spherical and homogeneous and the size of those particles is not appropriate for cell culture (Wang X, et al. Advanced healthcare materials. 2022 Apr;11(8):2102265. Micrometric sized spheres of extracellular matrix have been produced using a microfluidic system with incorporated heated platform. This system initiates the crosslinking process inside the microfluidic chip, however, the crosslinking kinetics of decellularized matrices by temperature are slow and usually need from 15 to 30 minutes to be completed (Lin Z, et al. ACS Biomaterials Science & Engineering. 2022 Mar 31;8(4):1644-55).

In this sense, the development of microspheres solving the above-mentioned technical problems and comprising natural polymers is therefore of great interest.

### DESCRIPTION OF THE INVENTION

In the first aspect, the present disclosure relates to a microsphere comprising: a) dECM; b) a natural biopolymer; and c) a crosslinking agent, wherein such microsphere has optimized viscosity to produce microspheres using microfluidics and electrospray methods.

In another aspect, the present disclosure relates to a composition comprising: i) a plurality of the microspheres of the present invention; and ii) at least biological cells and/or at least one biomolecule.

In another aspect, the present disclosure relates to the microspheres of the invention and/or the composition of the invention for use as medicament.

In another aspect, the present disclosure relates to the microspheres of the invention and/or the composition of the invention for use as biomolecule delivery.

In another aspect, the present disclosure relates to the microspheres of the invention and/or the composition of the invention for use in a method of wound healing or tissue regeneration or as a disease treatment material.

In another aspect, the present disclosure relates to a method of treating a medical condition which would benefit from tissue regeneration and/or cell transplantation in a subject in need thereof, comprising administering to the subject a therapeutically effective amount of a plurality of the microspheres and/or the composition described herein.

In another aspect, the present disclosure relates to a method of generating a microsphere comprising: comprising a) dECM; b) a natural biopolymer and c) a crosslinking agent, wherein the method comprising:
i) Providing a first solution of 0.5% to 5% w/v of dECM, preferably the dECM is from adipose tissue; and a second solution that ranges from 0.5% to 5% w/v of natural biopolymer, preferably wherein the natural biopolymer is selected from the list consisting of: chitosan, alginate, gellan, xanthan gum, pectin, hydroxypropyl-methylcellulose and any derivatives thereof, preferably chitosan; and mixing the first and second solutions;
ii) Mixing the composition obtained in step i) with a third solution of 1 to 5% w/v crosslinking agent, preferably the crosslinking agent is an ionic crosslinker agent selected from the list consisting of: sodium tripolyphosphate (TPP), sodium citrate (CIT), sulfosuccinic acid (SSA), oxalic acid (OA), glutaraldehyde (GLA), epichlorohydrin (ECH), trimethylpropane triglycidyl ether (TTE), ethylene glycol diglycidyl ether (EGDE), and any derivatives thereof, preferably TPP;
iii) Incubate the composition obtained in step ii) to cause the crosslinking between the crosslinker agent and the dECM:natural biopolymer, to generate the microspheres of adECM:natural biopolymer:crosslinking agent.

Particularly useful in the present invention are electrospray or microfluidic methods for generating the microspheres.

### BRIEF DESCRIPTION OF THE DRAWINGS

To complete the description and in order to provide for a better understanding of the invention, a set of drawings is provided. Said drawings form an integral part of the description and illustrate aspects and embodiments disclosed herein, which should not be interpreted as restricting the scope of the invention, but just as an example of how the invention can be carried out. The drawings comprise the following figures:

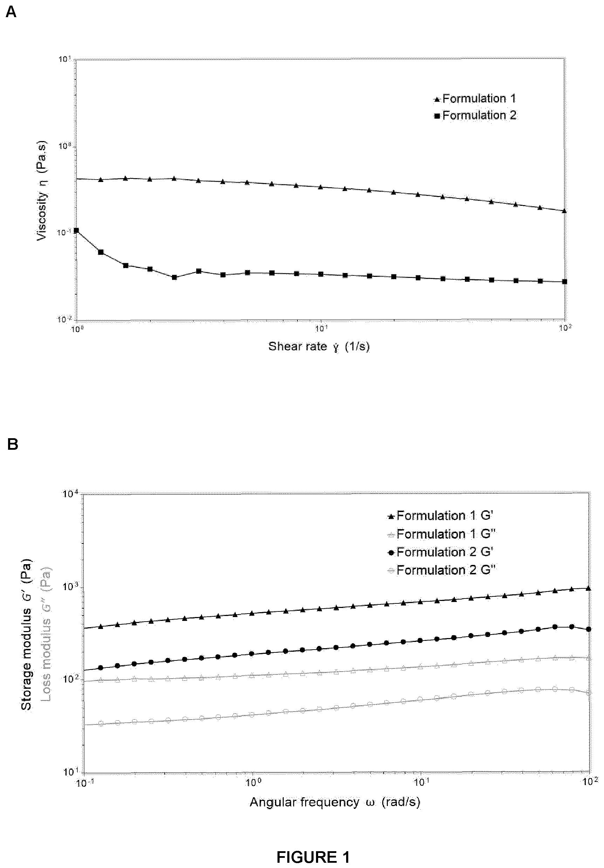

**Figure 1**. A) Dynamic viscosity of adipose dECM (adECM) solutions (1- 0.1 Pa·s at 1s-1 shear rate). B) Viscoelastic modulus of microspheres made of formulation 1 (microspheres produced by electrospray as disclosed in Example "*adECM Electrospray Microsphere Manufacturing*" included in the instant specification) and formulation 2 (microspheres produced by microfluidics as disclosed in Example *"adECM Microfluidic Microsphere Manufacturing").*

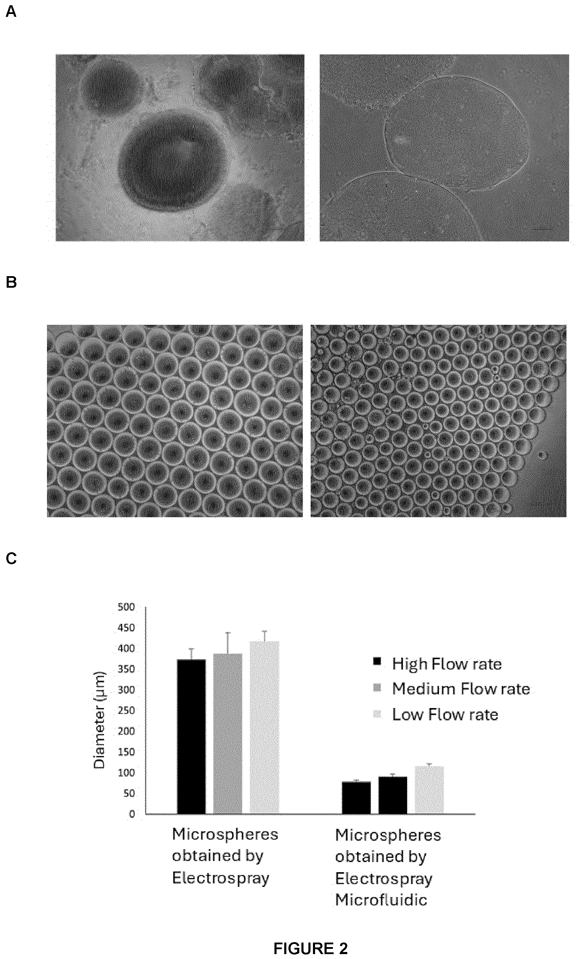

**Figure 2**. Bright-field images of microspheres produced by electrospray (A) as disclosed in Example "*adECM Electrospray Microsphere Manufacturing*" included in the instant specification; and microfluidics (B) as disclosed in Example "*adECM Microfluidic Microsphere Manufacturing*". Scalebar: 100 µm. (C) The bar graph presents the microsphere diameter using high (80 mL/min for fluorinated oil), medium (60 mL/min for fluorinated oil) and low (40 mL/min for fluorinated oil) flow rates. Microspheres obtained by electrospray methodology have higher viscosity than microspheres obtained by microfluidic methodology.

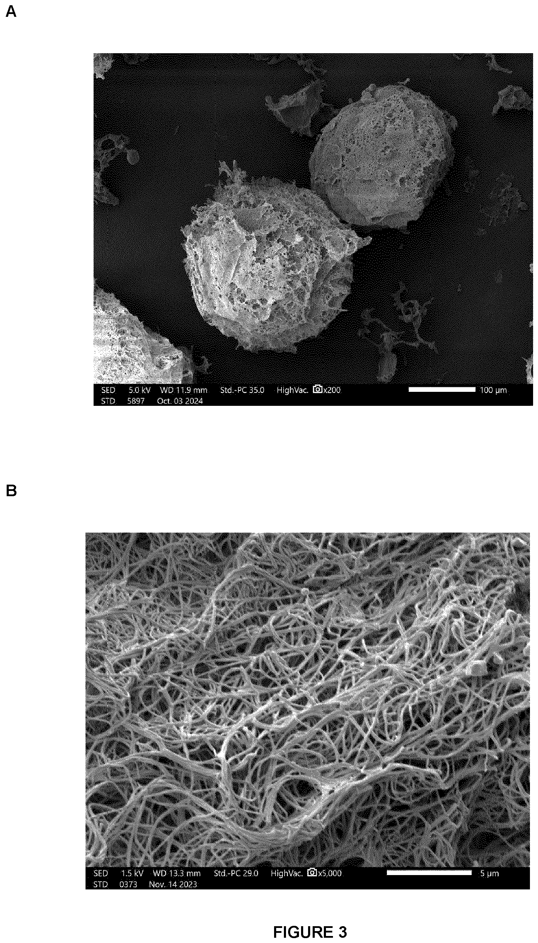

**Figure 3****.** Scanning electron microscope low (A) and high (B) magnification images of freeze-dried microspheres. Scalebar (A): 100 µm. Scalebar (B): 5 µm. This figure contains images of freeze-dried microspheres obtained by electrospray method where the spherical morphology and size can be appreciated. The high magnification images show the crosslinked fibers of adECM on the microsphere surface.

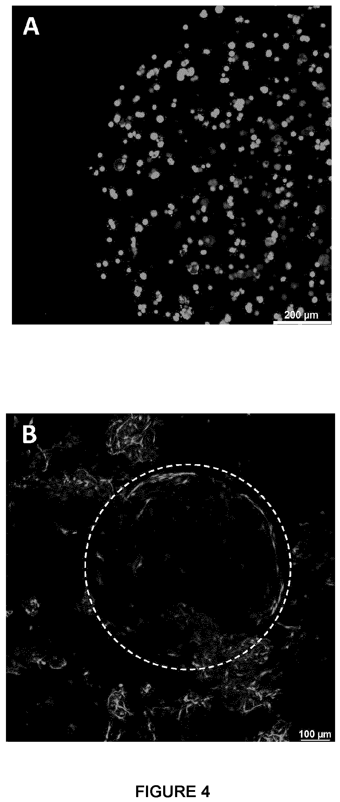

**Figure 4****.** Confocal images of BF-2 cells A) encapsulated in microspheres and B) cultured on the surface of microspheres stained with calcein (live cells, green colour) and propidium iodide (dead cells, red colour). Scalebar A): 200 µm. Scalebar B): 100 µm.

### DETAILED DESCRIPTION OF THE INVENTION.

The present inventors have now generated microspheres made of dECM, a natural biopolymer and a crosslinking agent, wherein cells and/or molecules (e.g. cytokines, growth factors, drugs etc.) and/or other molecules may be entrapped in and/or on these microspheres (**Figures 1 to 4**). These microspheres may serve as medicament and/or vehicles for protection and release of the cargo for various applications, such as tissue engineering, regeneration procedures and as a disease treatment material.

As the microspheres have optimized viscosity and a much greater surface area, their release can be much more precisely controlled and tailored to the specific desired usage. In addition, mass transfer of oxygen and nutrients is more efficient leading to improved cell viability. In fact, the surface of the microspheres of the present invention is not smooth, but porous and fibrous (**Figure 3**), with fibers between 5 and 100 nm in diameter.

According to the first aspect of the present invention, there is provided microsphere(s) comprising: a) dECM; b) a natural biopolymer and c) a crosslinking agent, wherein the percentage of the crosslinking agent versus the dECM and the natural biopolymer ranges from 0.1% to 10%, preferably from 0.5% to 7%, more preferably, from 0.5% to 7%, more preferably from 0.5 % to 5%.

The term "extracellular matrix (ECM)" as used herein, refers to a complex network of materials produced and secreted by the cells of the tissue into the surrounding extracellular space and/or medium and which typically together with the cells of the tissue impart the tissue its mechanical and structural properties. Generally, the ECM includes fibrous elements (particularly collagen, elastin, and/or reticulin), cell adhesion polypeptides (e.g., fibronectin, laminin and/or adhesive glycoproteins), and space-filling molecules [usually glycosaminoglycans (GAG), proteoglycans].

The term "decellularization" as used herein refers to a process by which a tissue is submitted to one or more treatments in order to maximize the removal of cells present in it, leaving only the ECM that is rich in structural proteins such as collagens, elastin, growth factors, and glycolipids.

In one embodiment, the dECM is from adipose tissue (adECM). In a more preferred embodiment, the adipose tissue is human or non-human mammal, preferably, human or porcine.

Methods of decellularizing ECM may be found in Cicuéndez, M., et al. Journal of Fungi. 2021a; 7(5) and WO2017114902A1, the contents of which are incorporated herein by reference. According to one embodiment of the present invention, the decellularization, preferably decellularization of adipose tissues, is carried out by:
a) Laminating the tissue, preferably the adipose tissue;
b) Treating the tissue resulting from step a) with a lipoprotein lipase either, at a concentration of 10-30 µ/100 mg for 39-49 hours at 32-42 °C or at a concentration of 45-55 µ/100 mg for 18-28 hours a 32-42 °C; and
c) Treating the adipose tissue resulting from step b) with a nuclease, either: at a concentration of 709-719 µ/mg for 67-77 hours at 32-42 °C or at a concentration of 1423-1433 µ/mg for 35-45 hours at 32-42 °C; or at a concentration of 1423-1433 µ/mg for 67-77 hours at 32-42 °C; for the adipose tissue to have a total of DNA content equal to or less than 50 ng/mg.

In a preferred embodiment, the method of decellularizing the tissue does not comprise the use of organic solvents.

In another preferred embodiment, the method of decellularizing the tissue further comprising a step between steps a) and b), wherein the adipose tissue resulting from step a) is treated with trypsin and triton-X100.

In another preferred embodiment, the method of decellularizing the tissue further comprises after steps a), b) and c), a washing step with buffer at room temperature, under vacuum and under stirring at 100-150 rpm, and wherein the washing step after step a) the buffer further comprises at least one antibiotic, at least one antimycotic and at least one protease inhibitor, in the washing step after step b) the buffer comprises at least one antibiotic, at least one antimycotic, at least one protease inhibitor and at least one lipase inhibitor and wherein the washing step after step c) the buffer further comprises at least one antibiotic, at least one antimycotic, at least one protease inhibitor and at least one nuclease inhibitor.

In another particular embodiment, the method of decellularizing further comprises a step d) comprising freezing or liofiphilizing and sterilizing the tissue resulting from step c). In another particular embodiment, the sterilization is carried out with ethylene dichloride. In another particular embodiment the sterilization is carried out with ultraviolet light. In another particular embodiment the method is carried out under aseptic conditions.

According to particular embodiments, more than 10%, 20%, 30%, 40%, 50%, 60%, 70%, 80%, 90% (w/v) of the microsphere is composed of adECM.

According to particular embodiments, more than 10%, 20%, 30%, 40%, 50%, 60%, 70%, 80%, 90% (w/v) of the microsphere is composed of a natural biopolymer.

According to particular embodiments, more than 10%, 20%, 30%, 40%, 50%, 60%, 70%, 80%, 90% (w/v) of the microsphere is composed of a crosslinking agent.

According to particular embodiments, the dECM-natural biopolymer and crosslinker agent are present in the microsphere in a ratio of about from 10:90 to about 60:40 by weight, preferably from about 20:80 to about 50:30 by weight, more preferably from about 30:70 to about 40:20; more preferably from about 40:60 to about 30:10.

According to particular embodiments, the crosslinker agent is present in the microsphere in a percentage from 0.5% to 5% by weight with respect to the dECM-natural biopolymer.

In another embodiment, the natural biopolymer is selected from the list consisting of: chitosan, alginate, gellan, xanthan gum, pectin, hydroxypropyl-methylcellulose, Polyhydroxyalkanoate, and any derivatives and/or combinations thereof. In a more preferred embodiment, the natural polymer is chitosan.

As used herein, the term "derivative" is a similar compound obtained by chemically changing a part of a compound but having the same or even better properties as the original compound. Thus, the derivatives of the natural polymers of the present invention refers to chemical modifications comprising the incorporation of different groups such as amino groups, aldehydes groups, carboxylic acids groups, amides groups, ketones groups, esters groups, alkoxy groups, sulphates groups, phosphates groups, or likes.

In another preferred embodiment, the crosslinking agent is an ionic crosslinker agent, preferably selected from the list consisting of: sodium tripolyphosphate (TPP), sodium citrate (CIT), sulfosuccinic acid (SSA), oxalic acid (OA), glutaraldehyde (GLA), epichlorohydrin (ECH), trimethylpropane triglycidyl ether (TTE), and ethylene glycol diglycidyl ether (EGDE). In a more preferred embodiment, the crosslinker agent is TPP.

As is shown herein, the natural biopolymer, such as chitosan, can be crosslinked with ionic bonds using highly biocompatible salts such as TPP. Together with adECM, the microspheres can be crosslinked in seconds without temperature need or perform a dual crosslinking if temperature is applied afterwards. The size of produced microspheres is appropriate and suitable for cell culture purposes, and the adECM provides excellent cell-adhesion properties and biochemical signaling. In fact, cells can be cultured either on top of the microspheres or encapsulated as a spherical 3D culture. The microspheres can be used for long-term culture of adherent cells on suspension bioreactors and are slowly degraded by cells and substituted and/or replaced by their own matrix.

In another preferred embodiment, the microsphere is between about 80 µm to about 700 µm in diameter, in the longest dimension. In a more preferred embodiment, the microsphere being between about from 80 µm to about 400 µm, from about 80 µm to about 110 µm, from about 250 µm to about 700 µm; or from about 300 µm to about 400 µm.

In another preferred embodiment, the microsphere has an optimized viscosity to be synthetized by microfluidics or electrospray methods. In this sense, the term "dynamic (shear) viscosity" of a fluid expresses its resistance to shearing flows. Viscosity influences droplet stability and size, thus, when the formulation to obtain the microsphere has high viscosity the microsphere obtained is large and stable but when the formulation to obtain de microsphere has a low viscosity, the microspheres obtained are small and not very stable. In addition, the viscoelastic modulus gives a value of the microspheres stiffness that is closely related to cellular response. Cells will culture and grow better in/on a viscoelastic surface (similar to that of biological tissue) than in/on rigid and not elastic surfaces. In this sense, the microspheres of the present invention has an storage modulus (hardness) which ranges from 100 Pa to 500 Pa.

As used herein, the term "about", when expressed as from "about" one particular value and/or "about" another particular value, also specifically contemplated and disclosed is the range from the one particular value and/or to the other particular value unless the context specifically indicates otherwise. It will be further understood that the endpoints of each of the ranges are significant both in relation to the other endpoint, and independently of the other endpoint unless the context specifically indicates otherwise. Finally, it should be understood that all of the individual values and subranges of values contained within an explicitly disclosed range are also specifically contemplated and should be considered disclosed unless the context specifically indicates otherwise. The foregoing applies regardless of whether in particular cases some or all of these aspects are explicitly disclosed.

Therapeutic compounds, agents that modify cellular activity and biomolecules can also be incorporated e.g. encapsulated in, attached to, coated on, embedded, or impregnated, into the microspheres.

As used herein, the term "biomolecule" can refer to organic and inorganic molecules (e.g., macromolecules or small molecules), such as proteins, enzymes, antibodies, nucleic acids (e.g., DNA, siRNA), drugs, and pharmaceutical compositions comprising one or more drugs. Exemplary compounds, agent and biomolecules that may be comprised into the microspheres of the present invention include but are not limited to those that promote cell adhesion (e.g. fibronectin, integrins), cell colonization, cell proliferation, cell differentiation, cell extravasation and/or cell migration. Thus, for example, the agent may be an amino acid, a small molecule chemical, a peptide, a polypeptide, a protein, a DNA, a RNA, a lipid and/or a proteoglycan.

Proteins that may be incorporated into the microspheres of the present invention include, but are not limited to extracellular matrix proteins, cell adhesion proteins, growth factors, cytokines, hormones, proteases and protease substrates. Thus, exemplary proteins include vascular endothelial-derived growth factor (VEGF), activin-A, retinoic acid, epidermal growth factor, bone morphogenetic protein, TGF-, hepatocyte growth factor, platelet-derived growth factor, TGFa, IGF-1and11, hematopoietic growth factors, heparin binding growth factor, peptide growth factors, erythropoietin, interleukins, tumor necrosis factors, interferons, colony stimulating factors, basic and acidic fibroblast growth factors, nerve growth factor (NGF) or muscle morphogenic factor (MMP), among others. The particular growth factor employed should be appropriate to the desired cell activity. The regulatory effects of a large family of growth factors are well known to those skilled in the art.

As well as, or instead of, the therapeutic compounds of agents described herein above, the present invention further contemplates incorporating (e.g. encapsulating in, attaching to, coating on, embedding and/or impregnating) cells into the microspheres described herein.

For cell incorporation, the cells are added to the solubilized adECM and/or to the microsphere of the invention.

The cells may be derived from any organism including, for example, mammalian cells, (e.g. human), plant cells, algae cells, fungal cells (e.g. yeast cells), prokaryotic cells (e.g. bacterial cells).

Exemplary cells include cardiac cells, neuronal cells, pancreatic cells, stem cells, liver cells, muscle cells, blood cells, immune cells, endothelial cells, epithelial cells, mesenchymal cells and circulating tumor cells.

According to a particular embodiment, the cells comprise stem cells - e.g. adult stem cells such as mesenchymal stem cells or pluripotent stem cells such as embryonic stem cells or induced pluripotent stem cells. The stem cells may be modified so as to undergo *ex vivo* differentiation.

According to another embodiment, the cells have been differentiated *ex vivo* from induced pluripotent stem cells.

According to a particular embodiment, the cells are preferably intact (i.e. whole), and preferably viable, although it will be appreciated that pre-treatment of cells, such as generation of cell extracts, or non-intact cells are also contemplated by the present invention.

The cells may be fresh, frozen or preserved in any other way known in the art (e.g. cryopreserved).

In another preferred embodiment of the present invention, the microspheres disclosed herein being formed by electrospraying techniques.

In another preferred embodiment of the present invention, the microspheres disclosed herein being formed by microfluidic techniques, preferably using a microfluidic chip.

In another aspect of the present invention, there is provided a composition comprising a plurality of the microspheres according to the present invention and at least biological cells and/or at least one compound, agent and/or molecules, preferably biomolecules, as disclosed herein.

In another preferred embodiment, the microspheres incorporate (e.g. encapsulated in, attached to, coated on, embedded or impregnated) said biological cells and/or at least one compound, agent, and/or molecules, preferably biomolecules, as disclosed herein.

In another preferred embodiment, the composition of the invention is a pharmaceutical composition that further comprises a pharmaceutically acceptable carrier.

As used herein, a "pharmaceutical composition" refers to the preparation of one or more of the microspheres described herein, as well as the composition disclosed herein, with other chemical components such as pharmaceutically suitable carriers and excipients. The purpose of a pharmaceutical composition is to facilitate administration of the microspheres and/or the composition comprising the microspheres to a subject.

Hereinafter, the term "pharmaceutically acceptable carrier" refers to a carrier oral diluent that does not cause significant irritation to a subject and does not abrogate the biological activity and properties of the administered compound. Examples, without limitations, of carriers are propylene glycol, saline, emulsions and mixtures of organic solvents with water.

Herein the term "excipient" refers to an inert substance added to a pharmaceutical composition to further facilitate administration of a compound. Examples, without limitation, of excipients include calcium carbonate, calcium phosphate, various sugars and types of starch, cellulose derivatives, gelatin, vegetable oils and polyethylene glycols.

Pharmaceutical compositions of the present invention may be manufactured by processes well known in the art, e.g., by means of conventional mixing, dissolving, granulating, dragee-making, levigating, emulsifying, encapsulating, entrapping or lyophilizing processes.

The microspheres of the present invention and the composition of the present invention may be used as medicament, preferably for treating any disorder associated with tissue degeneration. In a more preferred embodiment, the microspheres and the composition of the invention may be used in a method of wound healing or tissue regeneration or as a disease treatment material in any disorder associated with tissue degeneration.

In another aspect, the microspheres and the composition of the present disclosure can serve as a platform for delivery of one or more therapeutic compounds, agents and molecules to a target site (e.g., a tissue in need of repair or regeneration, such as diseased or dysfunctional tissue) *in vitro, ex vivo* and/or *in vivo.*

In another aspect of the present invention, there is provided a method of treating any disorder associated with tissue degeneration by the administration of a therapeutically effective amount of the microspheres or the composition of the present invention to a subject in need thereof.

As used herein, a therapeutically effective dose is an amount sufficient to effect a beneficial or desired clinical result, which dose could be administered in one or more administrations. According to one embodiment, a single administration is employed. The injection can be administered on any site in which tissue regeneration is required. Determination of an effective dose is typically effected based on factors individual to each subject, including, for example, weight, age, physiological status, medical history, and parameters related to the particular disorder to be treated. One skilled in the art would be able to determine the amount and number of cells comprised in the microspheres or in the composition of the present invention that would constitute an effective dose, and the optimal mode of administration thereof without undue experimentation.

In another aspect, methods are provided for generating microspheres comprising a) dECM, as disclosed herein; b) a natural biopolymer as disclosed herein; and c) a crosslinking agent as disclosed herein. In a preferred embodiment, the microsphere being formed by electrospraying techniques. In another preferred embodiment, the microspheres being formed by microfluidic techniques.

In another aspect, methods are provided for generating microspheres comprising a) dECM; b) a natural biopolymer and c) a crosslinking agent, wherein the method comprising:
i) Providing a first solution of 0.5% to 5% w/v of dECM, preferably the dECM is from adipose tissue; and a second solution that ranges from 0.5% to 5% w/v of natural biopolymer, preferably wherein the natural biopolymer is selected from the list consisting of: chitosan, alginate, gellan, xanthan gum, pectin, hydroxypropyl-methylcellulose and any derivatives thereof, preferably chitosan; and mixing the first and second solutions;
ii) Mixing the composition obtained in step i) with a third solution of 1 to 5% w/v crosslinking agent, preferably the crosslinking agent is an ionic crosslinker agent selected from the list consisting of: TPP, CIT, SSA, OA, GLA, ECH, TTE, EGDE, and any derivatives thereof, preferably TPP;
iii) Incubate the composition obtained in step ii) to cause the crosslinking between the crosslinker agent and the dECM-natural biopolymer, to generate the microspheres of adECM-natural biopolymer-crosslinking agent.

In a preferred embodiment, the first solution of dECM is provided at a concentration range of 1 % to 4% w/v, preferably at 1 % to 4% w/v; more preferably at 1 % to 3% w/v, more preferably at 1 to 2% w/v, even more preferable at a concentration of 1%.

In another preferred embodiment, the second solution of the natural biopolymer is provided at a concentration range of 1 % to 4% w/v, preferably at 1 % to 4% w/v; more preferably at 1% to 3% w/v, more preferably at 1 to 2% w/v, even more preferable at a concentration of 1%.

In a preferred embodiment, the third solution of the crosslinker agent is provided at a concentration range of 1 % to 4% w/v, preferably at 2% to 4% w/v; more preferably at 2% to 3% w/v, more preferably at 2% to 2,5% w/v, even more preferable at a concentration of 2%.

In another preferred embodiment, the method further comprises separating and isolating the microspheres, preferably by a method selected from the list consisting of filtration, decantation, centrifugation, electrostatic separation, air separation, sedimentation, dialysis and/or any combinations thereof. Any method known by the skilled person and useful for the purpose of this step can be used.

In another preferred embodiment, the method further comprises an additional step of adding at least one biological cell and/or a molecule, preferably wherein the molecule is a biomolecule, a drug, and a pharmaceutical composition comprising one or more drugs or any combinations thereof. The definitions mentioned in the present documents apply to all the aspects and embodiments of the present invention.

In another preferred embodiment, the method for generating the microspheres is selected from electrospray or microfluidic method.

In preferred embodiments, described in the Example below, microspheres of the present disclosure can be generated by electrospray of solubilized adECM. Microspheres size can be controlled by tuning electrospray voltage and syringe pump flow rate.

For the purposes of the electrospraying methods for generating microspheres according to the present disclosure, there is an aqueous phase comprising micronized adECM powder obtained as previously mentioned, digested at 2% w/v in 0.1 M HCl and pepsin 10% w/w. This solution is incubated for at least 24 hours at room temperature using magnetic shakers at a speed of at least 100 rpm. In addition, the aqueous phase also comprises a low molecular weight chitosan (50-190 kDa) solution prepared in 1M acetic acid at preferably 2% w/v. To obtain the final aqueous phase, the acidic adECM solution and the chitosan solution were introduced into the syringes. Both syringes were connected using luer-lock connectors and the contents of both, acidic adECM solution and chitosan solution, were mixed in a 1:1 ratio to obtain a final concentration of 1% adECM and 1% chitosan in the final aqueous phase solution.

Using a syringe pump, the acidic adECM solution, previously introduced into a syringe that is connected to a flexible duct containing a metal needle at the end, is extruded to the needle where a high voltage is applied that causes the fluid to be nebulized into microdroplets. The microdroplets are directed towards the collecting tray comprising biocompatible solutions that allow the spherical structure of the droplets to be maintained and/or to initiate cross-linking of the material. In a preferred embodiment, the biocompatible solution that allow the spherical structure of the droplets to be maintained and to initiate cross-linking of the material comprises the cross-linker agent, as above defined.

Once in the collection tray, the matrix microdroplets are polymerized by physical or chemical methods to obtain gelled microspheres according to the present invention. After that, the microspheres obtained are recovered by filtering steps, saline buffer washing, and decanting until a concentrated suspension of microspheres is obtained.

The concentration of the adECM solution, extrusion flow rate and applied voltage allow the size of the microdroplets to be controlled.

The electrospray technique produces microspheres in the range between 250 and 700 µm (**Figure 2A**). The differences in diameter are essentially due to the composition of the formulation/solution, as the dynamic viscosity and surface tension of the solutions vary. It has been observed that formulations with a low dynamic viscosity allow the formation of microspheres with a smaller diameter than those with a high dynamic viscosity.

For the production of microspheres of the present invention, preferably having about 700 µm in diameter, an extrusion flow rate of 0.07 mL/min is set in the syringe pump. The syringe loaded with the final aqueous phase solution is placed in a horizontal syringe pump (New Era Pump System Inc.) which is connected by a flexible PTFE tube to a metal needle of 0.6 mm diameter. This needle is oriented vertically and positioned perpendicular to a collecting tray at a distance of approximately 8 cm. The metal needle is connected to a Genvolt using power supply and a voltage of 19.7 kV is applied. The collecting tray contains a solution of TPP, preferably at a concentration of 2% w/v in distilled water that is placed on an orbital stirrer at a speed of 100 rpm. Thus, using these parameters, the final aqueous phase solution as above-mentioned is nebulized and directed towards the collecting tray. Once the entire syringe contents have been completely extruded, extrusion and voltage application are stopped, and the microspheres are incubated in the TPP solution for an additional 15 minutes. During this period, the chitosan chains form ionic bonds thanks to TPP salts, turning the microspheres into whitish solid structures. Once cross-linked, the contents of the collecting tray are poured into tubes, where the microspheres are decanted preferably by gravity. Once decanted, the TPP supernatant is removed and at least five washes, or as many as necessary, are carried out with distilled water to remove the excess cross-linking using the same decanting process. Once washed, the distilled water is removed, the microspheres can be resuspended in DMEM medium with 10% v/v fetal bovine serum and 1% v/v penicillin/streptomycin to be used for cell culture and differentiation or alternatively, can be freeze-dried to be preserved dry.

The microspheres obtained by the electrospraying methodology have usually a diameter between 80 µm to 700 µm, and as previously mentioned, the concentration of the adECM solution, extrusion flow rate and applied voltage allow the size of the microdroplets to be controlled.

In another embodiment, microspheres of the present disclosure can be generated by microfluidic method using a microfluidic chip whereby a pressurized flow controller drives an emulsion of adECM hydrogel precursor into emulsion with an oil-based carrier (e.g., mineral oil, fluorinated oil). Microspheres size can be adjusted by flow speed, surfactant concentration, chip dimensions, and degree of adECM digestion.

In preferred embodiments, polydimethylsiloxane (PDMS) chips manufactured by photolithography containing micrometric channels forming an X-shaped structure have been used. In the microfluidic method. The X-structure is designed for the entry of two streams of an oleic fluid that contact at the intersection with a stream of aqueous fluid in a perpendicular manner. The crossing of immiscible fluids allows the formation of microdroplets of the aqueous solution within the oleic solution that flow into the outlet channel. Fluids are pumped into the chip using a high-precision peristaltic pump.

In preferred embodiments, the aqueous phase is composed of a solution of adECM. As disclosed herein, the solution comprises micronized adECM powder obtained as disclosed in the examples disclosed herein, briefly, digested at, at least about 2% w/v in 0.1M HCl are prepared with pepsin at least 10% w/w. This solution is incubated for at least 24 hours at room temperature using magnetic shakers at a speed of about 100 rpm. In addition, the aqueous phase also comprises a low molecular weight chitosan (50-190 kDa) solution prepared in 1M acetic acid at preferably 2%w/v. To obtain the final aqueous phase, both solutions, the neutralized solution of adECM and the low molecular weight chitosan (50-190 kDa) solution are mixed at a 1:1 ratio.

In another preferred embodiment, the oleic phase is composed preferably of fluorinated oils with biocompatible surfactants to allow the correct dispersion of the microspheres. One skilled in the art would be able to determine other oils and biocompatible surfactants useful for the present invention without undue experimentation.

The microfluidic chips contain an outlet connected with a polytetrafluoroethylene (PTFE) tube to a reservoir containing the crosslinker agent, such as TPP at a concentration of 2% w/v. The adECM-Chitosan solution (aqueous solution) is injected by a peristaltic pump through a PTFE tubing at a rate of at least 10 µl/min and the oleic phase (fluorinated oil solution) at, at least 10 µl/min.

The microdroplets suspended in the oleic solution are collected at the outlet and incubated at a temperature that ranges from about 30 to 40 °C, preferably from about 33 to 37°C, more preferably from about 35-37°C, more preferably from about 36 to 37°C, and particularly preferably about 37°C, to initiate the polymerization of the adECM for about at least 30 minutes. The microspheres change from liquid to solid phase in the reservoir during an incubation period of at least 5 minutes. Once polymerized, additional steps are carried out to decant the microspheres by conventional methods known in the art such as filtering steps, and washing them with water, saline buffer or culture medium until the excess TPP and fluorinated oil are completely eliminated, and a concentrated oil-free microsphere suspension is obtained.

This design allows the size of the microspheres to be controlled by tuning the ratio of the oleic and aqueous flows, and this fact is easily performed by the one skilled in the art without undue experimentation. In addition, this method allows the use of low reagent volumes.

Microspheres of about 80 µm to 400 µm are obtained by this method. A higher viscosity formulation results in microspheres with a diameter in the range of about 300-400 µm (**Figure 2B**), while a low viscosity formulation results in microspheres with a diameter between about 80-110 µm. Using the same formulation, microfluidic manufacturing of microspheres allows size control based on the flow rate used. The use of high flow rates results in smaller sized microspheres than the use of low flow rates. In addition, it allows the use of low reagent volumes.

The composition of the microspheres obtained by anyone of the methods disclosed herein, is rich in structural proteins of the adECM, and generates fibrous surfaces with structures very similar to the fibers found in natural tissues that are composed primarily of structural collagen. This microstructure forms an ideal pattern, both in morphology and composition, to promote the formation of matrix cell bonds and allow both the anchoring of cells to the surface and division and migration in the microsphere.

**Figure 3** shows images of freeze-dried microspheres obtained by scanning electron microscopy of the lyophilized microspheres of the invention wherein the spherical morphology and size can be appreciated. As previously mentioned, the microspheres obtained in the present invention have fibrous surfaces with structures very similar to the fibers found in natural tissues that are composed primarily of structural collagen. The high magnification images show the crosslinked fibers of adECM on the microsphere surface.

As previously mentioned, the microspheres of the present invention can be useful as scaffold for cell culture and also further can incorporate (e.g. encapsulated in, attached to, coated on, embedded or impregnated) biological cells and/or at least one compound, agent, and/or molecules, preferably biomolecules, as disclosed in the present document.

adECM gel-based solid microspheres are highly hydrophilic and have a high-water content. Once in culture medium or buffered saline solutions, the microspheres precipitate by gravity, allowing a high density to accumulate at the bottom of culture plates and to be able to remove the supernatant easily. Thus, the precipitated microspheres are cultured with a cell suspension in full medium at the desired density and incubated for 2-24 hours to allow cell adhesion based on the specifications of each cell type. Once attached, coated, embedded or impregnated, the microspheres with cells can be recovered and used for bioreactor culture.

**Figure 4** presents an example of two types of cells seeding in microspheres. On the one hand, microfluidic fabrication allows the encapsulation of cells inside the microspheres, which provides a three-dimensional environment that mimics the physiological conditions of real tissues. The cells encapsulated in the microspheres manufactured by microfluidic method show high viability and grow within the microspheres forming spheroid-like cultures (**Figure 4A**). On the other hand, cells can be seeded on the surface of solid microspheres (**Figure 4B**) manufactured by electrospray or microfluidic methods. The cells have a high adherence to the surfaces of the microspheres, where they show a stellate morphology and a viability percentage greater than 95%. After being cultured for 7 days, the cells proliferate and invade the surface of the microspheres, showing a notable increase in cell biomass.

### EXAMPLES

The following are examples of the invention by means of assays carried out by the inventors, which evidence the effectiveness of the product of the invention. The following examples serve to illustrate the invention and must not be considered to limit the scope thereof.

### Porcine Adipose Tissue Decellularization

Porcine adipose tissue was harvested from a local food company (JAUCHA S.L., Navarra, Spain), cleaned, creamed using a beater and stored at -20 °C.

Tissues were decellularized following the methodology previously published by Cicuéndez, M.; *et al.* (Cicuéndez, M.; et al. Effects of Human and Porcine Adipose Extracellular Matrices Decellularized by Enzymatic or Chemical Methods on Macrophage Polarization and Immunocompetence. Int. J. Mol. Sci. 2021, 22, 3847). Briefly, the tissues were maintained at room temperature for defrosting, cleaned and creamed using a beater. Afterward, adipose tissues were homogenized on ice using Polytron PT3100 with two different rods at 12,000 rpm for 5 min. The homogenized tissue was centrifuged at 900 rpm for 5 min after the addition of 25 mL ultrapure water to favor the phase separation of lipids, which were discarded manually, keeping the protein pellet accumulated at the bottom of the container during the centrifugation.

After that, the protein pellets were treated by two different methods. In the first protocol, tissues were treated with 30 mL pure isopropanol (Merck Life Science SL, Madrid, Spain) overnight under orbital shaking at 100 rpm at room temperature. Afterwards, the material was thoroughly cleaned using PBS (PBS, Merck Life Science SL) supplemented with 1% (v/v) antibiotic-antimycotics solution (Gibco-BRL, Paisley, UK) and 125 µL protease inhibitors (Merck Life Sciences) and 0.1% (v/v) ammonium hydroxide (Merck Life Sciences) and finally treated with 1% (v/v) Triton X-100 (Merck Life Sciences) and 0.1% (v/v) ammonium hydroxide (Merck Life Sciences) for 36 h in an orbital shaker 100 rpm at room temperature. Then the materials were thoroughly cleaned, as previously described, and lyophilized until completely dry.

To create a fine-grained powder suitable for processing, the porcine decellularized adipose tissue was milled using a mixer mill (Retsch MM400, Biometa Tecnologia y Sistmas Parque Tecnológico de Asturias, Spain), preceded by freezing in a liquid nitrogen, and kept at 4°C in a vacuum desiccator.

### adECM Microfluidic Microsphere Manufacturing

Polydimethylsiloxane (PDMS) chips manufactured by photolithography containing micrometric channels forming an X-shaped structure have been used. The X-structure is designed for the entry of two streams of an oleic fluid that contact at the intersection with a stream of aqueous fluid in a perpendicular manner. The crossing of immiscible fluids allows the formation of microdroplets of the aqueous solution within the oleic solution that flow into the outlet channel. Fluids are pumped into the chip using a high-precision peristaltic pump. The aqueous phase is composed of a neutralized solution of adECM. The oleic phase is composed of fluorinated oils with biocompatible surfactants to allow the correct dispersion of the microspheres. The microdroplets suspended in the oleic solution are collected at the outlet and incubated at 37°C to initiate the polymerization of the adECM for about 30 minutes. The gelled microspheres of adECM are separated from the oleic phase by filtering steps, saline buffer washing, and decanting until a concentrated oil-free microspheres suspension is obtained. This design allows the size of the microspheres to be controlled by tuning the ratio of the oleic and aqueous flows. In addition, it allows the use of low reagent volumes.

For the purposes of the present example, the aqueous phase is composed of a neutralized solution of the micronized adECM powder obtained as previously disclosed digested at 2% w/v in 0.1M HCl and prepared with pepsin 10% w/w. This solution is incubated for 24 hours at room temperature using magnetic shakers at a speed of 100 rpm. In addition, the aqueous phase also comprises a low molecular weight chitosan (50-190 kDa) solution prepared in 1M acetic acid at preferably 2% w/v. To obtain the final aqueous phase, both solutions, the neutralized solution of adECM and the low molecular weight chitosan (50-190 kDa) solution are mixed at a 1:1 ratio.

The microfluidic chips were made with 100 µm in width and height. They contain an inlet for the mixture comprising the aqueous phase (both solutions above-mentioned, adECM:Chitosan) and an inlet for the oleic phase.

The oleic phase comprises fluorinated oil.

The microfluidic chips contain an outlet connected with a polytetrafluoroethylene (PTFE) tube to a reservoir containing the TPP crosslink at a concentration of 2% w/v. The adECM:Chitosan solution (aqueous solution) is injected by a peristaltic pump through a PTFE tubing at a rate of 10 µl/min and the oleic phase (fluorinated oil solution) at 10 µl/min.

The microdroplets suspended in the oleic solution are collected at the outlet and incubated at 37°C, to initiate the polymerization of the adECM for about at least 30 minutes. The microspheres change from liquid to solid phase in the reservoir during an incubation period of at least 5 minutes. Once polymerized, additional steps are carried out to decant the microspheres by conventional methods known in the art such as filtering steps, and washing them with water, saline buffer or culture medium until the excess TPP and fluorinated oil are completely eliminated, and a concentrated oil-free microsphere suspension is obtained. Thus, once washed, the microspheres can be resuspended in DMEM medium with 10% v/v fetal bovine serum and 1% v/v penicillin/streptomycin to be used for cell culture and/or differentiation or alternatively, can be freeze-dried to be preserved dry.

The diameter of the microspheres produced by microfluidics usually ranges from 80 to 400 µm. The diameter depends both on the formulations used and on the flow rate between the immiscible fluids. A formulation with higher viscosity results in microspheres with a diameter in the range of 300-400 µm (Figure 2B), while a formulation with a low viscosity result in microspheres with a diameter of between 80-110 µm. Using the same formulation, the manufacture of microspheres by microfluidics allows the size to be controlled based on the flow rate used. The use of high flow rates results in smaller bead sizes than the use of low flow rates.

The microspheres obtained by the above-mentioned methodology have about 365 ± 26 µm in diameter.

The data show in the examples are expressed as the mean ± standard deviation of a representative of three experiments carried out in triplicate. Statistical analysis was performed using the Statistical Package for the Social Sciences (SPSS) v. 22 software. Statistical comparisons were made by analysis of variance (ANOVA). A Scheffé test was used for post-hoc evaluations of the differences among groups. In all the statistical evaluations, p < 0.05 was considered as statistically significant.

The composition of the microspheres obtained is rich in structural proteins of the adECM, and generates fibrous surfaces with structures very similar to the fibers found in natural tissues that are composed primarily of structural collagen. This microstructure forms an ideal pattern, both in morphology and composition, to promote the formation of matrix cell bonds and allow both the anchoring of cells to the surface and division and migration in the microsphere.

**Figure 3** shows images of freeze-dried microspheres obtained by scanning electron microscopy of the lyophilized microspheres of the invention wherein the spherical morphology and size can be appreciated. As previously mentioned, the microspheres obtained in the present invention have fibrous surfaces with structures very similar to the fibers found in natural tissues that are composed primarily of structural collagen. The high magnification images (**Figure 3**) show the crosslinked fibers of adECM on the microsphere surface.

### Encapsulation of cells in microspheres obtained by microfluidics

The objective of the present example is to show as the microspheres of the present invention allow the encapsulation of cells, particularly MCF-7 cells from breast cancer.

MCF-7 breast cancer cells were cultured at a cell density of 1×10⁶ cells/mL for 24 h at 37 °C under a 5% CO₂ atmosphere in culture plates of 48-well culture plates coated with 100 µL of the microspheres of the invention obtained by microfluidic method, to reach a density of 1×10⁶ cells/mL. Briefly, the cell suspension is injected by a peristaltic pump into the adECM-Chitosan solution inlet at a rate of 10 µl/min (see above). On the other hand, the fluorinated oil is injected at 40 µl/min through the second inlet. The microspheres collected in the reservoir comprising TPP are cross-linked for 5 minutes and washed using complete culture medium using the decantation and resuspension steps described above. The microspheres were incubated in multiwell plates immersed in full culture medium. The culture medium was Dulbecco's Modified Eagle Medium (DMEM, Gibco-BRL, Paisley, UK) supplemented with 10% fetal bovine serum (FBS, Gibco-BRL, Paisley, UK), 1 mM L-glutamine (BioWhittaker Europe, Verviers, Belgium), penicillin (200 µg/mL, BioWhittaker Europe, Verviers, Belgium) and streptomycin (200 µg/mL, BioWhittaker Europe, Verviers, Belgium).

After 24 hours, the microspheres can be maintained in multiwell plates or transferred to bioreactors. The medium is changed every 2 days for 7 days. After this time, the complete medium is changed to a low serum differentiation medium to initiate myoblast differentiation into skeletal muscle fibers and incubated for an additional 7 days maintaining medium changes every 2 days. After 14 days, microspheres can be recovered and muscle differentiation analyzed using confocal microscopy or molecular techniques such as Western-Blot or qRT-PCR.

### Cell seeding on microspheres obtained by microfluidics

The objective of the present example is to show the usefulness of the microspheres of the present invention obtained by microfluidics, as above-mentioned, as spherical cell culture surfaces.

C2C12 mouse myoblasts were cultured at a cell density of 1×10⁶ cells/mL for 24 h at 37 °C under a 5% CO₂ atmosphere in culture plates of 96-well culture plates coated with 100 µL of the microspheres of the invention obtained by microfluidics as above-mentioned. The culture medium was Dulbecco's Modified Eagle Medium (DMEM, Gibco-BRL, Paisley, UK) supplemented with 10% fetal bovine serum (FBS, Gibco-BRL, Paisley, UK), 1 mM L-glutamine (BioWhittaker Europe, Verviers, Belgium), penicillin (200 µg/mL, BioWhittaker Europe, Verviers, Belgium) and streptomycin (200 µg/mL, BioWhittaker Europe, Verviers, Belgium). After that time, the cells are attached to the surface of the microspheres, and these can be monitored in multi-well plates over time or transferred to bioreactors.

The medium is changed every 2 days for 7 days. After this time, the complete medium is changed to a low serum differentiation medium to initiate myoblast differentiation into skeletal muscle fibers and incubated for an additional 7 days maintaining medium changes every 2 days. After 14 days, microspheres can be recovered and muscle differentiation analyzed using confocal microscopy or molecular techniques such as Western-Blot or qRT-PCR.

### adECM Electrospray Microsphere Manufacturing

The present example describes the fabrication of the microspheres of the present invention by electrospraying.

Acidic adECM solutions have been used for the manufacture of the microspheres of the present invention by electrospray technique. An acidic adECMx solution is introduced into a syringe that is connected to a flexible duct containing a metal needle at the end. This needle is connected to a high-voltage source and oriented perpendicular to a metal collecting tray. The collecting tray contains biocompatible solutions that allow the spherical structure of the droplets to be maintained and/or to initiate cross-linking of the material.

Using a syringe pump, the acidic adECM solution is extruded to the needle where a high voltage is applied that causes the fluid to be nebulized into microdroplets. The microdroplets are directed towards the collecting tray comprising an oleic solution, thus they maintain the spherical morphology.

Once in the collection tray, the matrix microdroplets are polymerized by physical or chemical methods to obtain gelled microspheres according to the present invention. After that, the microspheres obtained are recovered by filtering steps, saline buffer washing, and decanting until a concentrated suspension of microspheres is obtained.

The concentration of the adECM solution, extrusion flow rate and applied voltage allow the size of the microdroplets to be controlled.

The electrospray technique produces microspheres in the range between 250 and 700 µm (**Figure 2A**). The differences in diameter are essentially due to the composition of the formulation/solution, as the dynamic viscosity and surface tension of the solutions vary. It has been observed that formulations with a low dynamic viscosity allow the formation of microspheres with a smaller diameter than those with a high dynamic viscosity.

For the purposes of the present example, there is an aqueous phase comprising micronized adECM powder obtained as previously mentioned, digested at 2% w/v in 0.1 M HCl and pepsin 10% w/w. This solution is incubated for 24 hours at room temperature using magnetic shakers at a speed of 100 rpm. In addition, the aqueous phase also comprises a low molecular weight chitosan (50-190 kDa) solution prepared in 1M acetic acid at preferably 2% w/v. To obtain the final aqueous phase, 1.5 mL of the acidic adECM solution and 1.5 mL of chitosan solution were introduced into 3 mL syringes. Both syringes were connected using luer-lock connectors and the contents of both, acidic adECM solution and chitosan solution, were mixed in a 1:1 ratio to obtain a total volume of 3 mL and a final concentration of 1% adECM and 1% chitosan in the final aqueous phase solution.

For the production of microspheres having about 700 µm in diameter, an extrusion flow rate of 0.07 mL/min is set in the syringe pump. The syringe loaded with the final aqueous phase solution is placed in a horizontal syringe pump (New Era Pump System Inc.) which is connected by a flexible PTFE tube to a metal needle of 0.6 mm diameter. This needle is oriented vertically and positioned perpendicular to a collecting tray at a distance of approximately 8 cm. The metal needle is connected to a Genvolt using power supply and a voltage of 19.7 kV is applied. The collecting tray contains a solution of 2% w/v TPP in distilled water that is placed on an orbital stirrer at a speed of 100 rpm. Thus, using these parameters, the final aqueous phase solution as above-mentioned is nebulized and directed towards the collecting tray. Once the entire syringe contents have been completely extruded, extrusion and voltage application are stopped, and the microspheres are incubated in the TPP solution for an additional 15 minutes. During this period, the chitosan chains form ionic bonds thanks to TPP salts, turning the microspheres into whitish solid structures. Once cross-linked, the contents of the collecting tray are poured into 50 ml tubes, where the microspheres are decanted by gravity. Once decanted, the TPP supernatant is removed and at least 5 washes, or as many as necessary, are carried out with 20 ml of distilled water to remove the excess cross-linking using the same decanting process. Once washed, the distilled water is removed, the microspheres can be resuspended in DMEM medium with 10% v/v fetal bovine serum and 1% v/v penicillin/streptomycin to be used for cell culture and differentiation or alternatively, can be freeze-dried to be preserved dry.

The microspheres obtained by the electrospraying methodology usually have a diameter between 80 µm to 700 µm.

The composition of the microspheres obtained is rich in structural proteins of the adECM and generates fibrous surfaces with structures very similar to the fibers found in natural tissues composed primarily of structural collagen (Figure 3). This microstructure forms an ideal pattern, both in morphology and composition, to promote the formation of matrix cell bonds and allow both the anchoring of cells to the surface and division and migration in the microsphere.

### Cell seeding on microspheres obtained by electrospray

The objective of the present example is to show the usefulness of the microspheres of the present invention obtained by electrospray, as above-mentioned, as spherical cell culture surfaces.

Cell suspensions of C2C12 mouse myoblasts are prepared at a density of 1×10⁶ cells/ml. 300 µl of that said C2C12 mouse myoblasts suspension is seeded on the microspheres and incubated at 37°C with 5% CO2 for 24 hours in culture plates of 48-well culture plates comprising the microspheres. The culture medium was Dulbecco's Modified Eagle Medium (DMEM, Gibco-BRL, Paisley, UK) supplemented with 10% fetal bovine serum (FBS, Gibco-BRL, Paisley, UK), 1 mM L-glutamine (BioWhittaker Europe, Verviers, Belgium), penicillin (200 µg/mL, BioWhittaker Europe, Verviers, Belgium) and streptomycin (200 µg/mL, BioWhittaker Europe, Verviers, Belgium). After 24 hours, the cells are attached to the surface of the microspheres, and these can be monitored in multi-well plates over time or transferred to bioreactors.

The medium is changed every 2 days for 7 days. After this time, the complete medium is changed to a low serum differentiation medium to initiate myoblast differentiation into skeletal muscle fibers and incubate for an additional 7 days maintaining medium changes every 2 days. After 14 days, microspheres can be recovered and muscle differentiation analyzed using confocal microscopy or molecular techniques such as Western-Blot or qRT-PCR.

### Mechanical Properties of the Microspheres

adECM-based materials for the synthesis of microspheres can be formulated as viscous solutions for use in both electrospray and microfluidic methodologies. The viscosity of adECM-based materials solutions can be modified through changes in formulation thereof known by the skilled person. **Figure 1** shows the viscous properties of adECM pre-gelled solutions that are used for electrospray and microfluidic fabrication of the microspheres of the invention, and the storage and loss modulus of the resultant gels after crosslinking. As shown in **Figure 1A****,** both high and low viscosity solutions can be obtained depending on the formulation used. The formulation 1 relates to the formulation prepared for the microspheres produced by electrospray as disclosed in Example "*adECM Electrospray Microsphere Manufacturing*" included in the instant specification; and the formulation 2 relates to the formulation prepared for the microspheres produced by microfluidic as disclosed in Example "*adECM Microfluidic Microsphere Manufacturing*" included in the instant specification).

Viscous solutions act as fluids that can be easily extruded through conduits with a millimeter to micrometer diameter. Once the microdroplets are formed, they are transitioned from liquid to solid phase by using chemical crosslinkers, such as TPP as above-mentioned. In this way, solid and water-insoluble microspheres based on ECM are obtained.

The hardness or viscoelasticity of the microspheres can be modified by changes in the formulation of ECM-based solutions. Figure 1B shows higher storage modules than loss modules for both formulations. These results indicate that the materials have a more solid and less fluid behavior after the crosslinking process, thus confirming that solid microspheres are obtained after the manufacturing processes by electrospray and microfluidics.

On the other hand, an example of two formulations showing a difference of up to two times in the storage module is shown. In addition, formulations for the synthesis of the microspheres can be adapted to have culture surfaces that meet the biological specifications of each cell.

## Claims

1. A microsphere comprising: a) decellularized tissue extracellular matrix (dECM); b) a natural biopolymer and c) a crosslinking agent, wherein the dECM-natural biopolymer and crosslinker agent are present in the microsphere in a ratio of about from 10:90 to about 60:40 by weight, and wherein, preferably, the dECM is from adipose tissue.

2. The microsphere according to claim 1, wherein the crosslinker agent is present in the microsphere in a percentage about from 0.5% to 5% by weight with respect to the dECM and the natural biopolymer.

3. The microsphere according to any one of claims 1 to 2, wherein the natural biopolymer is selected from the list consisting of: chitosan, alginate, gellan, xanthan gum, pectin, hydroxypropyl-methylcellulose and any derivatives thereof, preferably wherein the natural biopolymer is chitosan.

4. The microsphere according to any one of claims 1 to 3, wherein the crosslinking agent is an ionic crosslinker agent, preferably selected from the list consisting of: sodium tripolyphosphate (TPP), sodium citrate (CIT), sulfosuccinic acid (SSA), oxalic acid (OA), glutaraldehyde (GLA), epichlorohydrin (ECH), trimethylpropane triglycidyl ether (TTE), ethylene glycol diglycidyl ether (EGDE), and any derivatives thereof, preferably the crosslinking agent is TPP.

5. The microsphere according to any one of claims 1 to 4, wherein the microsphere has an average diameter of about 80 µm to about 700 µm, preferably from 80 µm to 400 µm, from 80 µm to 110 µm, from 250 µm to 700 µm; or from 300 µm to 400 µm.

6. The microsphere according to any one of claims 1 to 5, encapsulating in, attaching to, coating on, embedding and/or impregnating at least one cell, preferably, wherein the cell is selected from the group consisting of: a cardiac cell, a neuronal cell, a pancreatic cell, a stem cell, a liver cell, a muscle cell, a blood cell, an immune cell, endothelial cell, epithelial cell, mesenchymal cell, and circulating tumor cell.

7. The microsphere according to any one of claims 1 to 6, encapsulating in, attaching to, coating on, embedding and/or impregnating at least one molecule, preferably wherein the molecule is a biomolecule, a drug, and a pharmaceutical composition comprising one or more drugs.

8. The microsphere according to any one of claims 1 to 7, being obtained by electrospray or by microfluidic techniques.

9. A composition comprising: i) a plurality of the microspheres of any one of claims 1 to 8; and ii) at least a biological cell and/or at least one molecule, preferably wherein the molecule is a biomolecule, a drug, and a pharmaceutical composition comprising one or more drugs or any combinations thereof.

10. The microsphere according to any one of claims 1 to 8, or the composition according to claim 9, for use as a medicament.

11. The microsphere according to any one of claims 1 to 8, or the composition according to claim 9, for use in a method of cell and/or molecule delivery; wound healing or tissue regeneration; or as a disease treatment material.

12. Method of generating microspheres comprising a) dECM; b) a natural biopolymer and c) a crosslinking agent, wherein the method comprising:
i) Providing a 0.5% to 5% w/v solution of dECM, preferably the dECM is from adipose tissue; and a second solution of 0.5% to 5% w/v natural biopolymer, preferably wherein the natural biopolymer is selected from the list consisting of: chitosan, alginate, gellan, xanthan gum, pectin, hydroxypropyl-methylcellulose and any derivatives thereof, preferably chitosan; and mixing the first and second solutions;
ii) Mixing the composition obtained in step i) with a third solution of 1% to 5% w/v crosslinking agent, preferably the crosslinking agent is an ionic crosslinker agent selected from the list consisting of: TPP, CIT, SSA, OA, GLA, ECH, TTE, EGDE, and any derivatives thereof, preferably TPP;
iii) Incubate the composition obtained in step ii) to cause the crosslinking between the crosslinker agent and the dECM-natural biopolymer, to generate the microspheres of dECM-natural biopolymer-crosslinking agent.

13. The method according to claim 12, further comprises separating and isolating the microspheres, preferably by a method selected from the list consisting of filtration, decantation, centrifugation, electrostatic separation, air separation, sedimentation, dialysis and/or any combinations thereof.

14. The method according to any one of claims 12 or 13, further comprises an additional step of adding at least one biological cell and/or a molecule, preferably wherein the molecule is a biomolecule, a drug, and a pharmaceutical composition comprising one or more drugs or any combinations thereof.

15. The method according to any one of claims 13 to 14 wherein the method is selected from electrospray or microfluidic method.
